# EUROPÄISCHE PATENTSCHRIFT

(11) **EP 0 748 866 B1**
(45) Veröffentlichungstag und Bekanntmachung des Hinweises auf die Patenterteilung: **16.08.2001**
(21) Anmeldenummer: 96109293.9
(22) Anmeldetag: 11.06.1996
(51) Int. Cl.: C12M 1/40, C12Q 1/26, C12Q 1/00

(54) **Verfahren und Mittel zur gleichzeitigen kolorimetrischen und elektrochemischen Messung eines Analyten**
Method and means for simultaneous colorimetric and electrochemical determination of an analyte
Méthode et moyens pour la détermination colorimétrique et électrochimique simultanées d'un analyte

(30) Priorität: 13.06.1995 DE 19521019
(43) Veröffentlichungstag der Anmeldung: 18.12.1996
(73) Patentinhaber: Roche Diagnostics GmbH, 68298 Mannheim (DE)
(72) Erfinder: Hoenes, Joachim, Dr., 64673 Zwingenberg (DE); Kotzan, Holger, Dr., 68526 Ladenburg (DE); Unkrig, Volker, Dr., 68526 Ladenburg (DE); Marquant, Michael, 68309 Mannheim (DE)

(56) Entgegenhaltungen:
- EP-A- 0 094 161
- EP-A- 0 433 854
- EP-A- 0 441 222
- DE-A- 3 247 894
- DE-A- 4 311 464

## Beschreibung

Die Erfindung betrifft ein Verfahren und ein Mittel zur gleichzeitigen kolorimetrischen und elektrochemischen Bestimmung eines Analyten mit einem oxidierenden Enzym.

Enzymatische Oxidationen ermöglichen in der Analytik den Nachweis und die Bestimmung von Substanzen in verschiedenen Probenmaterialien. Hierbei wirkt ein oxidierendes Enzym in Gegenwart eines die Elektronen der Oxidationsreaktion aufnehmenden Elektronenakzeptors auf ein ansprechendes Enzymsubstrat ein. Bei kolorimetrischen Nachweisreaktionen ist der Elektronenakzeptor ein Chromogen, das bei Reduktion direkt oder zusammen mit einer anderen Substanz Farbe bildet oder seine Farbe verändert und so die Anwesenheit des Enzymsubstrates anzeigt. Kolorimetrische Nachweisreaktionen können photometrisch oder auch vorteilhafterweise visuell ausgewertet werden und sind insbesondere bei niederen Analytkonzentrationen vorteilhaft. Hohe Analytkonzentrationen über 10⁻⁴M erzeugen im allgemeine schwer zu messende hohe Extinktionsänderungen von über 1 in Lösung und lassen sich insbesondere bei Remissionsmessungen auf Trockentestträgern schwer messen.

Für oxidierende Enzyme, das heißt für Oxidasen und Dehydrogenasen sind eine große Anzahl von künstlichen Elektronenakzeptoren bekannt. Beispiele können zum Beispiel in EP-A-0 330 517 gefunden werden.

Eine weitere Möglichkeit der Bestimmung eines Analyten mit einem oxidierenden Enzym ist die elektrochemische Messung, bei der eine Elektrode selbst als Elektronenakzeptor der Elektronen des Enzyms dient und ein entsprechender Strom gemessen wird. Ein solches Verfahren ist beispielsweise in EP-A-0 127 958 beschrieben. In EP-A-0 441 222 ist ein elektrochemisches Bestimmungsverfahren offenbart, bei dem neben dem Analyten eine Oxidoreduktase und eine reduzierbare Substanz zugegen sind und wobei nach der Re-Oxidation der reduzierten Substanz eine zweite Substanz entsteht Elektrochemische Messungen eignen sich insbesondere für höhere Analytkonzentrationen da eine im wesentlichen lineare Konzentrations-/Strombeziehung besteht.

In EP-A-0 094 161 wird der Nachweis eines Analyten mit einer PQQ-abhängigen Dehydrogenase und einem Chromogen als Elektronenakzeptor beschrieben, wobei die Reduktion des Chromogens auch elektrochemisch beobachtet werden soll. Als Anleitung, wie dies praktischerweise durchgeführt werden soll, wird lediglich angegeben, daß die Konzentrationsänderung zwischen reduzierter und oxidierter Form des reduzierbaren Elektronenakzeptors potentiometrisch mitverfolgt werden soll. Eine gleichzeitige amperometrische Messung bei einer solchen Farbbildungsreaktion ist nicht möglich, da die Elektronen prinzipiell entweder zur Reduktion des Chromogens oder zur Reduktion der Elektrode benötigt werden aber nicht für beides zur Verfügung stehen.

Es bestand daher Bedarf nach einem kombinierten elektrochemischen und kolorimetrischen Messverfahren, bei dem sowohl durch Reduktion eines Chromogens Farbe gebildet wird, bei dem andererseits aber auch gleichzeitig eine von der entstehenden farbigen Substanz verschiedene Verbindung als Maß für die Menge des Analyten entweder amperometrisch oder potentiometrisch gemessen werden kann. Damit könnten die Vorteile der kolorimetrischen Messung mit denen einer elektrochemischen Messung kombiniert werden und sich gegenseitig ergänzen. Dies sollte vor allem eine gleichmäßige Genauigkeit über einen besonders großen Analytkonzentrationsbereich ergeben. Die Aufgabe wird gelöst durch ein Verfahren und ein Mittel, wie es in den Ansprüchen charakterisiert ist.

Gegenstand der Erfindung ist ein Verfahren zur gleichzeitigen kolorimetrischen und elektrochemischen Messung eines Analyten mit einem oxidierenden Enzym und einem Chromogen A, das bei Reduktion vom Enzym Elektronen übernimmt, dadurch gekennzeichnet, daß das Chromogen A nach Reduktion zu A' durch Kupplung mit einer Substanz BX zu einer farbigen Substanz A'B reagiert, deren Konzentration kolorimetrisch als Maß für die Anwesenheit oder Menge des Analyten gemessen wird und daß bei der Kupplungsreaktion eine elektrochemisch meßbare Gruppe X⁻ abgespalten wird, deren Konzentration elektrochemisch als Maß für die Menge des Analyten gemessen wird.

Weiter ist Gegenstand der Erfindung ein Mittel zur gleichzeitigen kolorimetrischen und elektrochemischen Messung eines Analyten enthaltend ein oxidierendes Enzym und ein Chromogen A, das bei Reduktion vom Enzym Elektronen übernimmt, dadurch gekennzeichnet, daß es neben dem Chromogen A eine Substanz BX enthält, wobei das Chromogen A fähig ist, nach Reduktion durch Kupplung mit der Substanz BX unter Abspaltung einer elektrochemisch meßbaren Gruppe X zu einer farbigen Substanz A'B zu reagieren und bei der Kupplungsreaktion eine elektrochemisch meßbare Gruppe X⁻ abzuspalten und daß es zusätzlich eine Sensorelektrode zur elektrochemischen Messung der Menge oder Konzentration der abgespaltenen Gruppe X⁻ enthält.

Als oxidierende Enzyme kommen alle Enzyme in Betracht, die einen Analyten unter Elektronenübertragung der Elektronen auf ein künstliches Substrat A oxidieren. Dabei muß der Elektronenübergang vom Enzym auf das künstliche Substrat A nicht direkt erfolgen, sondern kann auch über einen Mediator, zum Beispiel Phenazinmethosulfat oder Diaphorase, erfolgen. Unter einem oxidierenden Enzym werden Oxidoreduktasen, insbesondere Oxidasen, NAD- und PQQ-abhängige Dehydrogenasen verstanden, besonders bevorzugt sind Dehydrogenasen.

Unter dem Chromogen A werden erfindungsgemäß alle Substanzen verstanden, die nach Reduktion eine kupplungsfähige Verbindung A' bilden, die wiederum nach Kupplung mit einer weiteren Substanz eine farbige Verbindung bildet, deren maximale Absorptionsänderung im sichtbaren - oder nahen Infrarot - Bereich, das heißt, zwischen 450 und 950 mm liegt

Bevorzugt als kupplungsfähige reduzierte Verbindungen A' sind von aromatischen Verbindungen abgeleitete Iminoverbindungen, insbesondere Chinondiimine, wie sie in EP-A-O 620 283 beschrieben sind, auf die vollinhaltlich Bezug genommen wird. Diese werden durch Reduktion von aromatischen Nitrosoverbindungen gebildet.

Besonders bevorzugt sind Nitrosobenzolderivate der allgemeinen Formel I 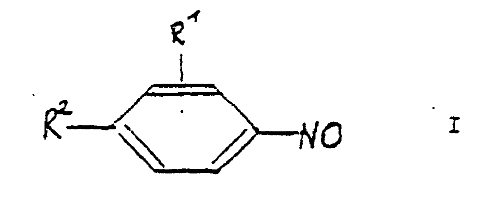
wobei R¹
   Wasserstoff, Hydroxy, Alkyl gegebenenfalls substituiert durch Hydroxy COOH, PO₃H₂, oder SO₃H, Alkoxy, Alkylthio, Aryloxy, Arylthio, Halogen, oder Amino, das gegebenenfalls ein oder mehrfach durch Alkyl, gegebenenfalls substituiert durch Hydroxy, PO₃H₂, Dialkylphosphinyl, SO₃ H oder CO₂H, substituiert ist, bedeutet
und R²
   eine Hydroxygruppe, , Alkoxy-, , Aryloxy-, Arylthio- oder Alkylthiogruppe bedeutet, wobei der Alkylrest gegebenenfalls seinerseits durch eine Hydroxygruppe, eine Alkoxygruppe, eine gegebenenfalls ein- oder mehrfach durch Alkyl substitutierte Aminogruppe, PO₃H₂, SO₃H oder CO₂H als solche oder in Salzform als Ammonium-, Alkali- oder Erdalkalisalze substituiert ist, oder eine Aminogruppe NR³ R⁴ bedeutet,
in der R³ und R⁴ gleich oder verschieden sein können, und Wasserstoff, eine Aryl- oder Alkylgruppe, die ihrerseits durch eine Hydroxy-, Alkoxy-, Hydroxyalkoxy-, eine gegebenenfalls hydroxysubstituierte Polyalkoxygruppe, PO₃H₂, SO₃H, COOH als solche oder in Salzform oder durch eine gegebenenfalls ein oder mehrfach durch Alkyl substituierte Aminogruppe substituiert sein kann, bedeutet, oder
in der R³ und R⁴ einen Alkylenrest darstellen können, der gegebenenfalls durch Sauerstoff, Schwefel oder Stickstoff unterbrochen ist, wobei Stickstoff durch einen Alkyl-, Hydroxyalkyl-, Hydroxyalkoxyalkyl-, Alkoxyhydroxyalkyl-, Alkoxycarbonylalkyl-, Dioxanylyl-alkyl- oder Polyalkoxyalkylrest substituiert ist, der seinerseits jeweils gegebenenfalls im Alkylteil durch einen Hydroxyrest substituiert sein kann, oder
   wenn R¹ in ortho-Stellung zu NR³R⁴ steht, R³ oder R⁴ auch zusammen mit R¹ einen Alkylenrest darstellen kann.

Hierbei bedeutet Halogen Fluor, Chlor, Brom oder Jod. Fluor und Chlor sind besonders bevorzugt.

Alkyl in Alkyl, Alkoxy oder Alkylthio bedeutet einen Kohlenwasserstoffrest mit 1 - 6 Kohlenstoffatomen, Reste mit 1 - 3 Kohlenstoffatomen sind besonders bevorzugt. Die für Alkyl vorstehend gegebene Definition trifft auch für den Alkylteil in Hydroxyalkyl-, Dialkylaminoalkyl-, Hydroxyalkoxyalkyl-, Alkoxyalkyl-, Polyalkoxyalkyl-, Alkoxy-hydroxyalkyl- und Dioxanylyl-alkylresten zu. Ein Dioxanylyl-alkylrest ist ein Rest, bei dem ein Dioxanringsystem an einen Alkylrest gebunden ist. Vorzugsweise handelt es sich um ein 1,4-Dioxanringsystem, d. h.

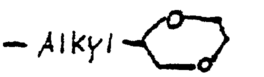

Ein Polyalkoxyalkylrest ist ein Rest -Alkyl- (Alkoxy)ₙ-Alkoxy
mit n = 1 - 10. Bevorzugt ist n = 1 - 4. Besonders bevorzugt ist n = 1 - 3. Ein Alkylenrest ist eine geradkettige oder verzweigte - vorzugsweise geradkettige -, gesättigte oder ungesättigte - vorzugsweise gesättigte-, Kohlenwasserstoffkette aus 2 - 5, vorzugsweise 2 - 4 C-Atomen, mit zwei freien Bindungsstellen.

Aryl in Aryl- und Aralkylresten ist ein 6 bis 10 Kohlenstoffatome zählendes aromatisches Ringsystem, wobei Phenyl bevorzugt ist.

Ammoniumsalze sind solche, die das Ammoniumion NH₄ + enthalten oder solche, die ein- oder mehrfach durch Alkyl-, Aryl- oder Aralkylreste substituierte Ammoniumkationen enthalten.

Alkalisalze sind vorzugsweise solche des Lithium, Natriums oder Kaliums. Erdalkalisalze sind vorzugsweise solche des Magnesiums oder Calciums.

Bevorzugte Reste R¹ sind Wasserstoff und Alkyl, insbesondere Wasserstoff.

Bevorzugte Reste R² sind Alkoxyreste und die Aminogruppe NR³R⁴.

In der Bedeutung eines von R¹ und R³ durch Sauerstoff, Schwefel oder Stickstoff unterbrochenen Alkylenrestes ist der durch Einbeziehung des Stickstoffatomes der allgemeinen Formel I gebildete Morpholin- bzw. Thiomorpholin- bzw. Piperazinrest bevorzugt. Der Piperazinrest ist besonders bevorzugt.

In der Bedeutung eines von R¹ und R³ gebildeten Alkylenrestes ist der durch Einbeziehung des aromatischen Ringes der allgemeinen Formel I gebildete Indolin- oder 1,2,3,4-Tetrahydrochinolinrest bevorzugt.

Als Salz eines erfindungsgemäßen Nitrosoanilinderivates der allgemeinen Formel I werden insbesondere solche starker Säuren, insbesondere Mineralsäuren, wie Salzsäure, Schwefelsäure, Salpetersäure, Phosphorsäure bevorzugt. Ganz besonders bevorzugt sind Hydrocnloride, das sind Salze der Salzsäure.

Bevorzugte Nitrosoverbindungen der allgemeinen Formel I sind:
N,N'- Bis-(2-hydroxyethyl)-p-nitrosoanilin
N,N'- Dimethyl -p-nitrosoanilin
N,N'- Diethyl -p-nitrosoanilin
N-Methyl-N'-(4-nitrosophenyl)-piperazin
N-(2-hydroxyethyl)-5-nitrosoindolin
2,4-Dimethoxy-nitrosobenzol
N,N'-Bis-(2-methoxyethyl))-4-nitrosoanilin
N-(4-Nitrosophenyl)-morpholin
N-(2,2-diethoxy-ethyl)N'-(4-nitrosophenyl) -piperazin
p-Nitrosophenol
3-Methoxy-4-nitrosophenol

Unter den elektronenreichen heteroaromatischen Nitrosoverbindungen, deren aromatisches Ringsystem so elektronenreich ist, daß ein externer +M-Substituent für die Nitroso-/Oxim-Tautomerie und für die Iminbildung entbehrlich ist, sind besonders mit einer Nitrosogruppe substituierte Pyrazolone, Pyrazole und insbesondere nitrososubstituierte Pyrazoloverbindungen, wie sie zum Beispiel in Ullmann's Enzyclopedia of Industrial Chemistry 5th ed., Vol A 20, Seite 72 bis 74, beschrieben sind, im erfindungsgemäßen Verfahren geeignet. Bevorzugt sind dabei die 3-Nitrosopyrazolo-Verbindungen der allgemeinen Formel II.

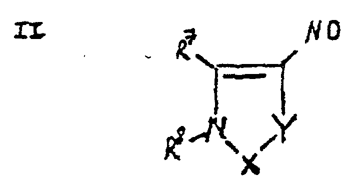

Bekannt sind diese Verbindungen zum großen Teil als Vorstufe zur präparativen Synthese der entsprechenden 3-Aminopyrazoloverbindungen aus der Europäischen Patentanmeldung EP-A-O 433 854. In der Formel II bedeutet:
- X-Y: NR⁵-CO oder N=CR⁶
- R⁵: Wasserstoff, Alkyl gegebenenfalls substituiert durch Hydroxy, Carboxy, SO₃H, PO₃H₂, Dialkylphosphinyl
- R⁶: Wasserstoff, Alkyl, Alkenyl, Alkoxy, Alkyithio, Aryl, Arylthio, Aralkyl, gegebenenfalls jeweils substituiert durch Hydroxy, Carboxy, SO₃H, PO₃H₂, ein Salz eines dieser Säurereste oder/und Alkoxycarbonyl; oder
Amino, das gegebenenfalls durch einen oder zwei gegebenenfalls einen oder mehrere Hydroxy-, Carboxy- oder/und Alkoxycarbonylreste tragende Alkylreste substituiert ist wobei, wenn Amino durch 2 Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann, oder Amino, das gagebenenfalls durch ein oder zwei Acylgruppen, Alkoxy- oder/und Aralkoxycarbonylgruppen, H₂N-CO, Alkyl-, Aralkyl- oder/und Arylcarbamoylgruppen substituiert ist; oder Carboxy, Alkoxycarbonyl, Carboxamido oder Halogen, und
- R⁷: Alkyl, Thioalkyl oder Aralkyl, gegebenenfalls substituiert durch Hydroxy, Carboxy, SO₃H oder PO₃H₂ oder Amino, das gegebenenfalls durch eine oder zwei Alkylgruppen substituiert ist, die wiederrum durch Hydroxy, Carboxy, SO₃H, Dialkylphosphinyl oder PO₃H₂ substituiert sein können,
wobei mindestens R6 und/oder R7 eine Aminogruppe darstellt, und
- R⁸: eine Alkyl oder Aralkylgruppe, die gegebenenfalls durch Hydroxy, Carboxy, SO₃H oder PO₃H₂ substituiert sein kann
oder wobei
R⁷ und R⁸ zusammen eine gesättigte oder ungesättigte Kette mit 3 oder 4 Gliedern aus Stickstoffatomen oder aus Kohlenstoffatomen und gegebenenfalls einem oder mehreren Stickstoff- oder Schwefelatomen, wobei Kohlenstoffatome gegebenenfalls substituiert sind durch Alkyl, Alkoxy, Alkylthio, Hydroxy, Aralkyl, Aryl, Carboxy, Carboxamido, Alkoxycarbonyl, Cyano, Halogen, Amino, das gegebenenfalls durch einen oder zwei gegebenenfalls einen oder mehrere Hydroxy-, Carboxy- oder/und Alkoxycarbonylreste tragende Alkylreste substituiert ist, und wobei Stickstoffatome, die nicht über eine Doppelbindung gebunden sind, durch Alkyl oder Aralkyl gegebenenfalls durch Hydroxy, SO₃H, PO₃H₂, Carboxy oder Dialkylphosphinyl substituiert, substituiert sind oder zwei benachbarte Kettensubstituenten gegebenenfalls eine Alkylengruppe bilden, die ihrerseits gegebenenfalls mit Aryl substituiert oder anelliert ist
sowie gegebenenfalls entsprechende tautomere Formen und deren Salze
Hierbei bedeutet "Alkyl" - auch in Alkylthio-, Dialkyl-phosphinyl-, Alkylcarbamoyl- und Aralkylresten - einen geradkettigen oder verzweigten Alkylrest mit 1-6, vorzugsweise 1-4 C-Atomen. Beispiele sind die Methyl-, Ethyl-, Propyl-, iso-Butyl- oder tert.-Butylgruppe.

Wenn eine Aminogrupe durch 2 Alkylreste substituiert ist, können diese Reste auch so zu einem Ring geschlossen sein, daß sie insgesamt einen durch ein Stickstoffatom unterbrochenen Ring darstellen. Bevorzugt sind hierbei solche Aminogruppen, die einen insgesamt 5- oder 6-gliedrigen Ring darstellen und der seinerseits gegebenenfalls durch Sauerstoff, Schwefel oder Stickstoff unterbrochen ist. Besonders bevorzugt ist der Morpholinorest.

"Alkoxy" - auch in Alkoxy- und Aralkoxycarbonylresten - steht für einen geradkettigen oder verzweigten Alkoxyrest mit 1 - 6, vorzugsweise 1 - 4 C-Atomen. Beispiele sind die Methoxy-, Ethoxy-, Propyloxy-, iso-Butyloxy- oder tert.-Butyloxygruppe.

"Aryl" - auch in Arylcarbamoylgruppen - bezeichnet einen Kohlenstoffaromaten- oder Heteroaromatenrest, vorzugsweise einen solchen mit 6 - 10 Ring-Atomen, insbesondere die Phenyl- und die Naphthylgruppe, die zusätzlich noch durch Alkyl, Alkoxy oder/und Halogen substituiert sein können. Besonders bevorzugt ist der Phenylrest.

Ein "Aralkyl"-Rest - auch in einer Aralkylcarbamoylgruppe -bedeutet einen Rest, bei dem eine wie vorstehend definierte Alkylgruppe durch einen wie zuvor charakterisierten Arylrest substituiert ist. Bevorzugt ist die Benzylgruppe.

Ein "Aralkoxy"-Rest, beispielsweise in Aralkoxycarbonylgruppen, bezeichnet einen Rest, bei dem eine wie vorstehend definierte Alkoxygruppe durch einen wie zuvor charakterisierten Arylrest substituiert ist. Bevorzugt ist die Benzyloxygruppe.

"Halogen" steht für die Reste Fluor, Chlor, Brom und Jod. Fluor und Chlor sind bevorzugt.

Eine Acylgruppe bezeichnet einen Carbonsäurerest, der Alkyl-, Aralkyl- oder Arylreste enthalten kann. Bevorzugt sind Acetyl-, Phenylacetyl- oder Benzoylreste.

Unter einer Alkylengruppe wird eine geradkettige oder verzweigte, gesättigte oder ungesättigte Kohlenwasserstoffkette aus 3 - 5, vorzugsweise 3 oder 4 C-Atomen mit zwei freien Bindungsstellen verstanden.

Beispiele sind -CH₂-CH=CH-, 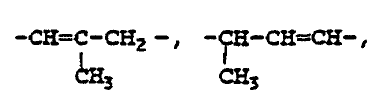 -(CH₂)₄- oder -CH=CH-CH=CH-.

Bevorzugt sind der Butadiendiylrest (-CH=CH-CH=CH-) und der Tetramethylenrest (-(CH₂)₄-).

Ein Alkenylrest ist ein geradkettiger oder verzweigter Kohlenwaserstoffrest aus 2-5 C-Atomen mit mindestens einer Doppelbindung. Bevorzugt ist beispielsweise der Vinylrest. Unter einer Dialkylphosphinylgruppe wird der Rest 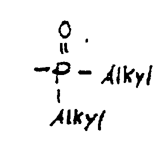 verstanden, wobei Alkyl die zuvor gegebene Bedeutung hat. Bevorzugt ist der Dimethylphosphinylrest.

Als Salze von SO₃H, PO₃H₂ und Carboxyresten können Alkali- oder Erdalkalisalze oder Ammoniumsalze eingesetzt werden. Unter Alkalisalzen werden Lithium-, Natrium-, Kalium-, Rubidium- und Cäsiumsalze verstanden, wobei Lithium-, Natrium- und Kaliumsalze, vor allem aber Natrium- und Kaliumsalze bevorzugt sind. Erdalkalisalze sind solche des Berylliums, Magnesiums, Calciums, Strontiums oder Bariums. Bevorzugt sind Magnesium- und Calciumsalze, wobei Calciumsalze besonders bevorzugt sind. Als Ammoniumsalze können solche des unsubstituierten Ammoniumions, NH₄⁺, Verwendung finden. Es ist aber auch möglich, solche Ammoniumsalze einzusetzen, bei denen das Ammoniumion durch 1 - 4 Alkyl-, Aryl- oder Aralkylreste substituiert ist. Für diese Reste gelten die zuvor gegebenen Definitionen, wobei als Alkylrest Methyl, Ethyl und n-Propyl, als Arylrest die Phenylgruppe und als Aralkylrest die Benzylgruppe besonders bevorzugt sind.

Als Carboxamidorest wird der Rest CONH₂ verstanden, aber auch solche Reste, bei denen die Aminogruppe durch ein oder zwei gegebenenfalls einen oder mehrere Hydroxy-, Carboxy- oder/und Alkoxycarbonylreste tragende Alkylreste substituiert ist.

In den erfindungsgemäß eingesetzten Nitroso-Verbindungen der allgemeinen Formel II sind solche Verbindungen bevorzugt, in denen R7 und R8 eine gesättigte oder ungesättigte Kette wie oben beschrieben bildet. Besonders bevorzugt ist dabei, wenn diese Kette ungesättigt ist und Doppelbindungselektronen und freie Stickstoffelektronenpaare der ungesättigten Kette in Konjugation zu der Doppelbindung oder zu dem Brücken-N-Atom der allgemeinen Formel II stehen, so daß ein anellierter aromatischer Ring resultiert.

Gegebenenfalls sind für eine Substanz der allgemeinen Formel II auch tautomere Formen möglich. Diese sollen ebenfalls als von der allgemeinen Formel II umfaßt gelten.

Erfindungsgemaß bevorzugt sind Nitrosoverbindungen der allgemeinen Formeln III bis XII. 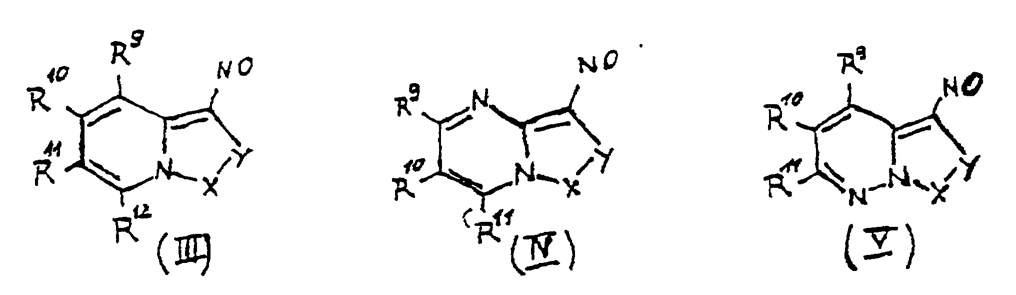 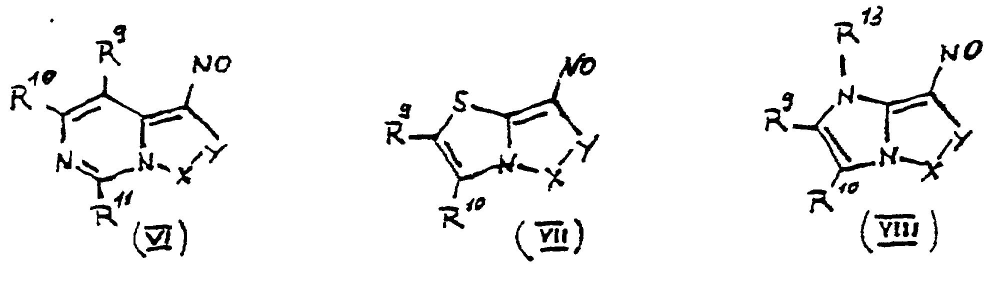 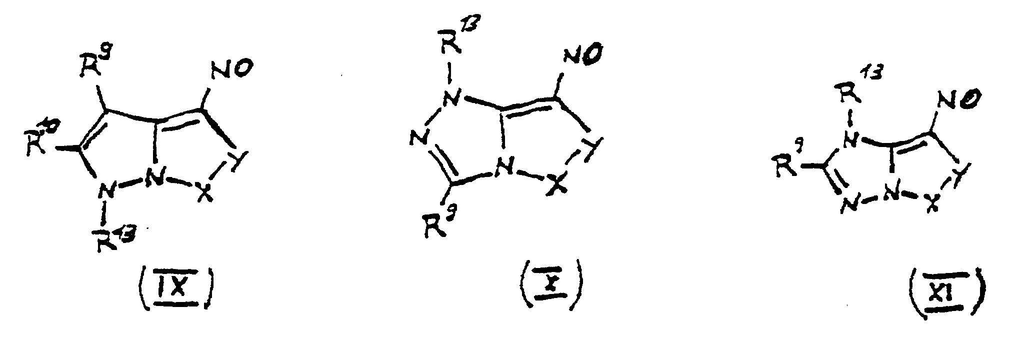 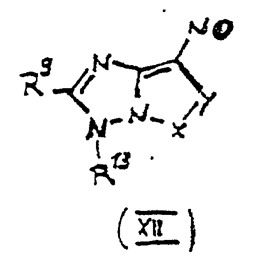 sowie gegebenenfalls entsprechende tautomere Formen und deren Salze.

Hierbei hat X-Y die gleiche Bedeutung wie zuvor beschrieben. R⁹, R¹⁰, R¹¹ und R¹², die gleich oder verschieden sind, für Wasserstoff, Hydroxy, Alkyl, Alkoxy, Alkylthio, Aralkyl, Aryl, Carboxy, Alkoxycarbonyl, Carboxamido, Cyano, Amino, das gegebenenfalls durch ein oder zwei gegebenenfalls einen oder mehrere Hydroxy-, Carboxy- oder/und Alkoxycarbonylreste tragende Alkylreste substituiert ist oder Halogen, wobei zwei benachbarte Reste gegebenenfalls eine Alkylengruppe bilden, die ihrerseits gegebenenfalls mit Aryl substituiert oder anelliert ist und R¹³ Alkyl oder Aralkyl darstellt, das gegebenenfalls durch Hydroxy, Carboxy, SO₃ H, PO₃H₂ oder Dialkylphosphinyl substituiert sein kann. Die Definitionen der Reste entsprechen den für die Substanzen der allgemeinen Formel II gegebenen.

Besonders bevorzugt für die erfindungsgemäße Verwendung sind Substanzen der allgemeinen Formeln III, IV, V, VII, VIII und IX, gegebenenfalls entsprechende tautomere Formen und deren Salze. Ganz besonders bevorzugt sind solche Substanzen, in denen X-Y die Bedeutung N=CR⁶ hat, wobei R⁶ die Bedeutung haben kann wie für die allgemeine Formel II angegeben.

Als hervorragend geeignet für die erfindungsgemäße Verwendung haben sich insbesondere die Verbindungen 3-Nitroso-2-methyl-pyrazolo-[1,5a]-pyridin, 3-Nitroso-pyrazolo-[1.5a]-pyridin und 3-Nitrosopyrazolo [3.2-c]-s-triazol und deren Salze, insbesondere das Hydrochlorid erwiesen.

Bevorzugt als Kupplersubstanz BX werden substituierte aromatische Verbindungen, insbesondere Anilin, Naphthylamin, Phenole und Naphthole mit einer leicht substituierbaren Gruppe verwendet. Als substituierbare, abspaltbare Gruppe werden bevorzugt Halogene, insbesondere Brom und Chlor oder eine SO3H-Gruppe verwendet

Das bevorzugte erfindungsgemäße Reaktionsprinzip ist demnach eine nucleophile aromatische Substitution der abspaltbaren Gruppe X der Verbindung BX durch die reduzierte Verbindung A'.

Bevorzugte Verbindungen BX sind 2,4,6-Trihalogen-3-Hydroxybenzolsäure,2,4-Dihalogen-1-Naphthol-1-Naphthol-4-Sulfonsäure, 4-Monohalogen-Aniline, -Phenole und -Naphthole.

Je nach Natur der abgespaltenen Gruppe X⁻ kann amperometrisch oder potentiometrisch die Konzentration dieser Gruppe gemessen werden. Ist die abgespaltete Gruppe ein Halogenidion, so wird vorteilhafterweise potentiometrisch gegen eine Ag/Ag-Halogenid-EIektrode oder Kalomel-Elektrode gemessen. Andere abspaltbare Gruppen, wie die SO3⁻ Gruppe können auch amperometrisch durch Oxidation an der Elektrode gemessen werden.

Gleichzeitig mit der elektrochemischen Messung der abgespalteten Gruppe X⁻ wird auch die gebildete Farbe des Kupplungsproduktes A'B kolorimetrisch gemessen. Dies kann visuell oder bevorzugt über ein Photometer erfolgen. Dabei ergänzen sich kolorimetrische und potentiometrische Messungen in idealer Weise, da insbesondere bei niederen Konzentrationen die kolorimetrische Messung von Vorteil ist, während bei höheren Konzentrationen die elektrochemische Messung eine genauere Bestimmung erlaubt. Zudem kann die Farbbildung dafür benutzt werden, das Funktionieren des elektrochemischen Detektionssystems zu kontrollieren.

Zur direkten amperometrischen Messung steht das Reaktionsgemisch mit einer inerten festen Elektrode (z.B. einer Platinelektrode) in Kontakt, die fähig ist, Elektronen von der abspaltbaren Gruppe aufzunehmen und die eine Elektrode einer Halb-Zelle bildet Die Halb-Zelle ist mit einer zweiten Elektrode leitfähig verbunden, an der eine Reduktionsreaktion stattfinden kann (z.B. eine Silber- / Silberchloridelektrode, Kalomelelektrode oder Platin/Ferrocyanid). Bevorzugt bildet eine Salzbrücke eine flüssige Verbindung zwischen den beiden Elektroden.

Zur potentiometrischen Messung der abgespaltenen Ionen X⁻ ist es vorteilhaft, ionensensitive Elektroden beispielsweise chlorid- oder bromidsensitive Elektroden zu verwenden. Diese sind in einer großen Bandbreite kommerziell erhältlich. Geeignete Elektrodenanordnungen und Meßverfahren sind dem Fachmann bekannt

Das erfindungsgemäße Verfahren wird bevorzugt in einer wäßrigen, nicht elektrochemisch aktiven Lösung durchgeführt. Zur Einhaltung eines für die Durchführung des Verfahrens geeigneten pH-Wertes, der sich insbesondere nach dem einzusetzenden Enzym richtet, enthält das erfindungsgemäße Mittel bevorzugt ein Puffersystem. Vorteilhafterweise stellt das Puffersystem der Testlösung einen pH-Wert zwischen 4 und 9, insbesondere zwischen 5 und 7 ein.

Das erfindungsgemaße Mittel kann in Form einer Lösung oder in trockener Form auf einem saugfähigen oder quellbaren Träger aufgezogen vorliegen. In Form einer Lösung enthält das Mittel vorzugsweise sämtliche für das Verfahren benötigten Reagenzien. Als Lösungsmittel kommen bevorzugt Wasser, aber auch Mischungen mit wasserlöslichen organischen Lösungsmitteln wie zum Beispiel, Methanol, Ethanol, Aceton oder Dimethylformamid in Frage. Aus Haltbarkeitsgründen kann es vorteilhaft sein, die für den Test benötigten Reagenzien auf zwei oder mehrere Lösungen zu verteilen, die erst bei der eigentlichen Untersuchung vermischt werden. Die Konzentration des eingesetzten künstlichen Elektronenakzeptors richtet sich nach der Konzentration des zu messenden Analyten.

Typische Konzentrationen für die durch das erfindungsgemaße Verfahren zu messenden Analyte sind 10⁻⁶ bis 10⁻² mol /l, insbesondere 10⁻⁵ bis 10⁻² mol/L Entsprechend sind typische Konzentrationen der eingesetzten künstlichen Elektronenakzeptoren A 10⁻⁴ bis 10⁻¹ mol /l. Die Konzentration des oxidierenden Enzyms richtet sich nach dessen Aktivität und der Konzentration des Analyten. Typische Werte für Enzymkonzentrationen sind 1 mU / ml bis 1 U/ml bei Küvettentests.

Die kupplungsfähige Verbindung BX wird mindestens im stöchiometrischen Verhältnis zur Verbindung A eingesetzt, bevorzugt in einem 1,5- bis 2-fachen Überschuß.

Das erfindungsgemäße Mittel kann auch in Form eines Teststreifens vorliegen. Solche Teststreifen sind in verschiedensten Ausführungsformen bekannt. In einem Teststreifen liegen die zur Durchführung des Bestimmungsverfahrens benötigten Reagenzien auf festen Trägerschichten vor. Als Trägerschichten kommen insbesondere saugfähige und / oder quellbare Materialien in Frage, die von der zu untersuchenden Probenflüssigkeit benetzt werden. In oder auf diesen Trägermaterialien liegen die Reagenzien in fester Form vor. Bei Aufgabe der Probenflüssigkeit auf den Teststreifen oder Eintauchen des Teststreifens in die Probenflüssigkeit bildet sich in den Streifen ein flüssiges Milieu aus, innerhalb dessen die Nachweisreaktion abläuft. Die zur Messung der abspaltbaren Gruppe X- benötigte Elektrode steht dabei in flüssigem leitfähigem Kontakt zu diesem flüssigen Milieu der Reaktionsmischung.

Die bevorzugten Konzentrationen der einzelnen Reagenzien auf Teststreifen betragen:
Analyt typischerweise 10⁻⁴ bis 10⁻¹ M
Chromogen A 10⁻³ bis 1 M
Kupplungsfähige Substanz BX 10⁻³ bis 1 M
Enzym 0,1 bis 100 U pro Testfeld

### Beispiel 1

### Glucosenachweis mittels Farbbildung und elektrochemischer Detektion

### Reaktionsgleichung

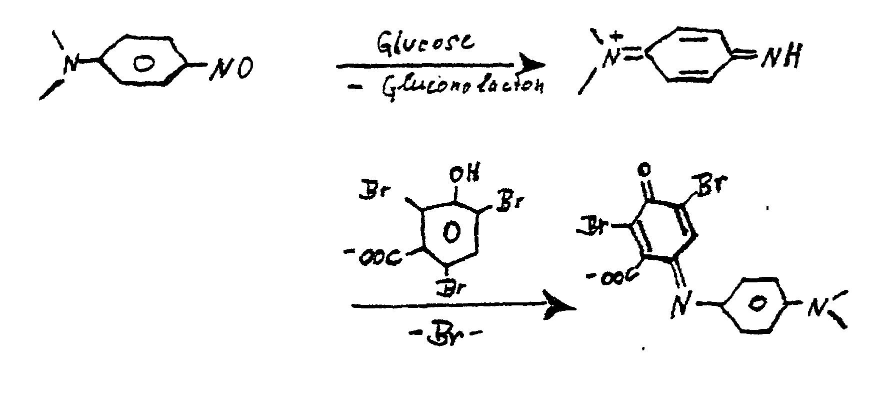

### Messansatz (Endkonzentration):

100 mM Citratpuffer pH 5,8
10 mM 2,4,6-Tribrom-3-Hydroxybenzoesäure
1 mM N,N-Bis-(2-Hydroxyethyl)-4-Nitrosoanilin
10 U/ml Glucose-Dye-Oxidoreduktase (PQQ-abhängige Glucosedehydrogenase)
0,1 M Natriumnitrat

Als Analyt wird Glucose in verschiedenen Konzentrationen zwischen 100 und 500 µM eingesetzt.

### Messung

### a) elektrochemische Messung:

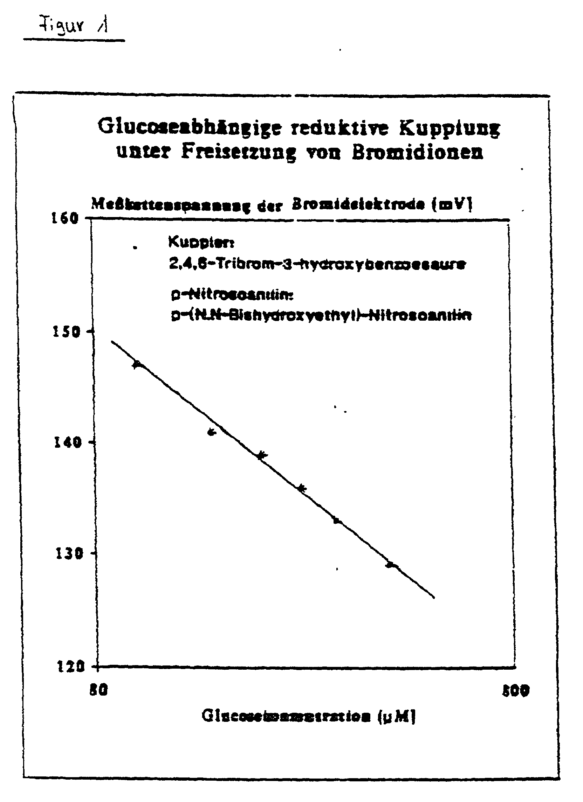

Die wahrend der reduktiven Kupplung des Chinondiimins A' mit 2,4,6-Tribrom-3-Hydroxybenzoesäure frei werdenden Bromidionen werden mittels einer ionensensitiven Elektrode der Firma Ingold, Typ 373-90-WTE-ISE-S7 in Verbindung mit einen Minivoltmeter der Firma Knick (Typ 763) gemessen. Das Ergebnis ist in Figur 1 dargestellt.

### b) kolorimetrische Messung

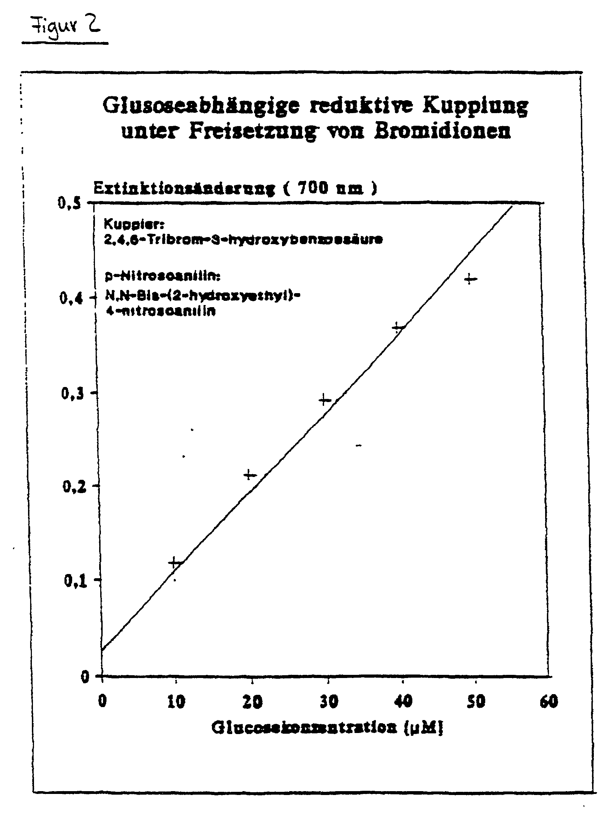

Parallel zur Zunahme der Bromidkonzentration (Abnahme der Messkettenspannung der bromidsensitiven Elektrode) mit zunehmender Glucosekonzentration wird die zunehmende Bildung eines grünen Farbstoffes A'B mit λ max. = 705 nm beobachtet (Figur 2).

## Patentansprüche

1. Verfahren zur gleichzeitigen kolorimetrischen und elektrochemischen Messung eines Analyten mit einem oxidierenden Enzym und einem Chromogen A das vom Enzym Elektronen übernimmt, dadurch gekennzeichnet, daß das Chromogen A eine elektronenreiche aromatische Nitrosoverbindung ist, die nach Reduktion zu einer kupplungsfähigen Iminoverbindung A' durch Kupplung mit einer Substanz BX zu einer farbigen Substanz A'B reagiert, deren Konzentration kolorimetrisch als Maß für die Anwesenheit oder Menge des Analyten gemessen wird und daß bei der Kupplungsreaktion eine elektrochemisch meßbare Gruppe X⁻ abgespalten wird, deren Konzentration elektrochemisch als Maß für die Menge des Analyten gemessen wild.

2. Verfahren gemäß Anspruch 1, dadurch gekennzeichnet, daß das Chromogen eine Verbindung der Formel I darstellt 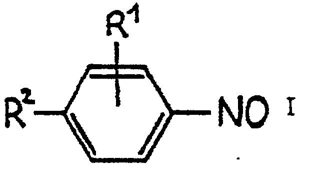
wobei R¹
Wasserstoff, Hydroxy, Alkyl, Alkyl substituiert durch Hydroxy, COOH, PO₃H₂, oder SO₃H, Alkoxy, Alkylthio, Aryloxy, Arylthio, Halogen, Amino oder Amino, das ein oder mehrfach durch Alkyl oder Alkyl substituiert durch Hydroxy, PO₃H₂, Dialkylphosphinyl, SO₃H oder CO₂H substituiert ist, bedeutet
und R²
eine Hydroxygruppe, Alkoxy-, Aryloxy-, Arylthio- oder Alkylthiogruppe bedeutet, wobei der Alkylrest unsubstituiert oder seinerseits durch eine Hydroxygruppe, eine Alkoxygruppe, eine Aminogruppe oder eine ein- oder mehrfach durch Alkyl substituierte Aminogruppe, PO₃H₂, SO₃H oder CO₂H als solche oder in Salzform als Ammonium-, Alkali- oder Erdalkalisalze substituiert ist, oder eine Aminogruppe NR³R⁴bedeutet, in der R³ und R⁴ gleich oder verschieden sein können, und Wasserstoff, eine Aryl- oder Alkylgruppe, die ihrerseits durch eine Hydroxy-, Alkoxy-, Hydroxyalkoxy-, eine Polyalkoxygruppe oder eine substituierte Polyalkoxygruppe, PO₃H₂, SO₃H, COOH als solche oder in Salzform oder durch eine Aminogruppe oder eine ein oder mehrfach durch Alkyl substituierte Aminogruppe substituiert sein kann, bedeutet, oder in der R³ und R⁴ einen Alkylenrest darstellen können oder einen Alkylenrest, der durch Sauerstoff, Schwefel oder Stickstoff unterbrochen ist, wobei Stickstoff durch einen Alkyl-Hydroxyalkyl-, Hydroxyalkoxyalkyl-, Alkoxyhydroxyalkyl-, Alkoxycarbonylalky-, Dioxanylyl-alkyl- oder Polyalkoxyalkylrest substituiert ist, der seinerseits jeweils unsubstituiert oder im Alkylteil durch einen Hydroxyrest substituiert sein kann, oder wenn R¹ in ortho-Stellung zu NR³R⁴ steht, R³ oder R⁴ auch zusammen mit R¹ einen Alkylenrest darstellen kann.

3. Verfahren gemäß Anspruch 1, dadurch gekennzeichnet, daß das Chromogen eine Verbindung der allgemeinen Formel II darstellt, in der 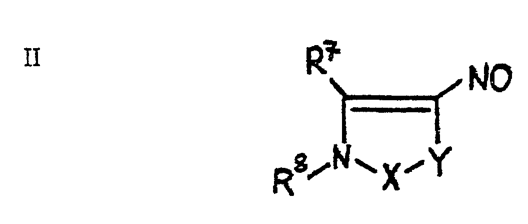
X-Y NR⁵-CO oder N=CR⁶
R⁵ Wasserstoff, Alkyl, Alkyl substituiert durch Hydroxy, Carboxy, SO₃H, PO₃H₂, Dialkylphosphinyl
R⁶ Wasserstoff, Alkyl, Alkenyl, Alkoxy, Alkylthio, Aryl, Arylthio, Aralkyl, die jeweils durch Hydroxy, Carboxy, SO₃H, PO₃H₂, ein Salz eines dieser Säurereste oder/und Alkoxycarbonyl substituiert sein können; oder Amino oder Amino, das durch einen oder zwei Alkylreste, die einen oder mehrere Hydroxy-, Carboxy- oder/und Alkoxycarbonyireste tragen können, substituiert ist, wobei, wenn Amino durch 2 Alkylreste substituiert ist, diese Reste auch zu einem Ring geschlossen sein können, der außer durch das N-Atom der Aminogruppe auch durch Sauerstoff, Schwefel oder ein weiteres Stickstoffatom unterbrochen sein kann, oder Amino oder Amino, das durch ein oder zwei Acylgruppen, Alkoxy- oder/und Aralkoxycarbonylgruppen, H₂N-CO, Alkyl-, Aralkyl- oder/und Arylcarbamoylgruppen substituiert ist; oder Carboxy, Alkoxycarbonyl, Carboxamido oder Halogen, und
R⁷ Alkyl, Thioalkyl oder Aralkyl, das substituiert sein kann durch Hydroxy, Carboxy, SO₃H oder PO₃H₂ oder Amino oder Amino, das durch eine oder zwei Alkylgruppen substituiert ist, die wiederrum durch Hydroxy, Carboxy, SO₃H, Dialkylphosphinyl oder PO₃H₂ substituiert sein können, . wobei mindestens R⁶ und/oder R⁷ eine Aminogruppe darstellt, und
R⁸ eine Alkyl oder Aralkylgruppe, die durch Hydroxy, Carboxy, SO₃H oder PO₃H₂ substituiert sein kann bedeutet.

4. Verfahren gemäß Anspruch 1-3, dadurch gekennzeichnet, daß die kupplungsfähige Substanz BX eine aromatische Verbindung mit einem leicht durch eine nudeophile aromatische Substitutionsreaktion substituierbaren Substituenten X darstellt.

5. Verfahren gemäß Anspruch 4, dadurch gekennzeichnet, daß BX ein Halogen- oder Sulfonsäure-substituiertes Phenol oder Naphthol oder Anilin oder Naphthylamin darstellt.

6. Verfahren gemäß Anspruch 5, dadurch gekennzeichnet, daß die kupplungsfähige Substanz BX 1-Naphthol-4-Sulfonsäure darstellt, die bei Kupplung eine SO₃H⁻-Gruppe abspaltet

7. Verfahren gemäß Anspruch 5, dadurch gekennzeichnet, daß die kupplungsfähige Substanz BX 2,4,6-Trihalogen-3-Hydroxybenzoesäure oder 2,4-Dihalogen-1-Naphthol oder 4-Monohalogenphenol oder -1-Naphthol darstellt, die ein Halogenidion abspaltet.

8. Verfahren gemäß Anspruch 6, dadurch gekennzeichnet, daß die Gruppe X⁻ potentiometrisch gemessen wird.

9. Verfahren gemäß Anspruch 7, dadurch gekennzeichnet, daß die Gruppe X⁻ amperometrisch gemessen wird.

10. Mittel zur gleichzeitigen kolorimetrischen und elektrochemischen Messung eines Analyten enthaltend ein oxidierendes Enzym und ein Chromogen A, das vom Enzym Elektronen übernimmt, dadurch gekennzeichnet, daß es neben dem Chromogen A, welches eine elektronenreiche aromatische Nitrosoverbindung darstellt, eine Substanz BX enthält, wobei das Chromogen A fähig ist, nach Reduktion durch Kupplung mit der Substanz BX unter Abspaltung einer elektrochemisch meßbaren Gruppe X⁻ zu einer farbigen Substanz A'B zu reagieren und daß das Mittel zusätzlich eine Sensorelektrode zur elektrochemischen Messung der Anwesenheit oder Konzentration der abgespaltenen Gruppe X⁻ enthält.

11. Mittel gemäß Anspruch 10, dadurch gekennzeichnet, daß die Substanz BX eine Phenol- oder Naphtholverbindung oder Anilin oder Napthylamin mit einer leicht substituierbaren, elektrochemisch meßbaren X⁻ Abgangsgruppe darstellt

12. Mittel gemäß Anspruch 11, dadurch gekennzeichnet, daß die Substanz BX 1-Naghthol-4-Sulfonsäure darstellt.

13. Mittel gemäß Anspruch 11, dadurch gekennzeichnet, daß BX 2,4-Dihalogen-1-Naphthol oder 4-Monohalogenphenol oder -1-Naphthol oder 2,4,6-Trihalogen-3-Hydroxybenzosäure ist.

14. Mittel gemäß Anspruch 10-12, dadurch gekennzeichnet, daß es zusätzlich einen Mediator für die Elektronenübertragung vom Enzym auf das Chromogen A enthält

15. Mittel gemäß Anspruch 10-14, dadurch gekennzeichnet, daß es zusätzlich eine optische Meßvorrichtung zur Bestimmung der Anwesenheit oder Konzentration der farbigen Substanz A'B enthält.

## Claims

1. Method for the simultaneous colorimetric and electrochemical measurement of an analyte using an oxidizing enzyme and a chromogen A that accepts electrons from the enzyme, wherein the chromogen A is an electron-rich aromatic nitroso compound which, after reduction to a coupling compound A', reacts by coupling with a substance BX to form a coloured substance A'B the concentration of which is measured colorimetrically as a measure for the presence or amount of the analyte and wherein an electrochemically measurable group X⁻ is cleaved off in the coupling reaction the concentration of which is measured electrochemically as a measure for the amount of the analyte.

2. Method as claimed in claim 1, wherein the chromogen is a compound of formula I 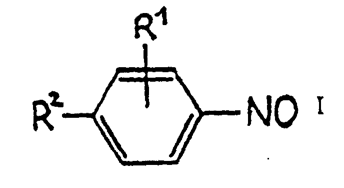
in which R¹
denotes hydrogen, hydroxy, alkyl, alkyl substituted by hydroxy, COOH, PO₃H₂, or SO₃H, alkoxy, alkylthio, aryloxy, arylthio, halogen, amino or amino which is substituted once or several times by alkyl or alkyl substituted by hydroxy, PO₃H₂, dialkylphosphinyl, SO₃H or CO₂H
and R²
denotes a hydroxy group, alkoxy, aryloxy, arylthio or alkylthio group in which the alkyl residue is unsubstituted or is itself substituted by a hydroxy group, an alkoxy group, an amino group or an amino group substituted once or several times by alkyl, or it denotes PO₃H₂, SO₃H or CO₂H as such or in a salt form as ammonium salts, alkali salts or alkaline earth salts or an amino group NR³R⁴, in which R³ and R⁴ can be the same or different and denote hydrogen, an aryl or alkyl group which in turn can be substituted by a hydroxy, alkoxy, hydroxyalkoxy, a polyalkoxy group or a substituted polyalkoxy group, PO₃H₂, SO₃H, COOH as such or in a salt form or can be substituted by an amino group or an amino group substituted once or several times by alkyl or in which R³ and R⁴ can represent an alkylene residue which is interrupted by oxygen, sulphur or nitrogen wherein nitrogen is substituted by an alkyl, hydroxyalkyl, hydroxyalkoxyalkyl, alkoxyhydroxyalkyl, alkoxycarbonylalkyl, dioxanylylalkyl or polyalkoxyalkyl residue each of which in turn can be unsubstituted or substituted in the alkyl moiety by a hydroxy residue or
if R¹ is in the ortho position relative to NR³R⁴, R³ or R⁴ can represent an alkylene residue also together with R¹.

3. Method as claimed in claim 1, wherein the chromogen represents a compound of the general formula II in which 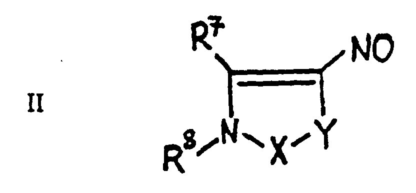
X-Y denotes NR⁵-CO or N=CR⁶
R⁵ denotes hydrogen, alkyl, alkyl optionally substituted by hydroxy, carboxy, SO₃H, PO₃H₂, dialkylphosphinyl
R⁶ denotes hydrogen, alkyl, alkenyl, alkoxy, alkylthio, aryl, arylthio, aralkyl each of which can be substituted by hydroxy, carboxy, SO₃H, PO₃H₂, a salt of one of these acid residues or/and alkoxycarbonyl; or amino or amino which is substituted by one or two alkyl residues that can carry one or several hydroxy, carboxy, or/and alkoxycarbonyl residues wherein if amino is substituted by 2 alkyl residues these residues can also be closed to form a ring that in addition to the N-atom of the amino group can also be interrupted by oxygen, sulphur or a further nitrogen atom or amino or amino which is substituted by one or two acyl groups, alkoxy or/and aralkoxycarbonyl groups, H₂N-CO, alkyl, aralkyl or/and arylcarbamoyl groups; or carboxy, alkoxycarbonyl, carboxamido or halogen and
R⁷ denotes alkyl, thioalkyl or aralkyl which can be substituted by hydroxy, carboxy, SO₃H or PO₃H₂ or amino or amino which is substituted by one or two alkyl groups which in turn can be substituted by hydroxy, carboxy, SO₃H, dialkylphosphinyl or PO₃H₂, wherein at least R⁶ and/or R⁷ represents an amino group and
R⁸ denotes an alkyl or aralkyl group which can be substituted by hydroxy, carboxy, SO₃H or PO₃H₂.

4. Method as claimed in claims 1-3, wherein the coupling substance BX represents an aromatic compound with a substituent X that can readily be substituted by means of a nucleophilic aromatic substitution reaction.

5. Method as claimed in claim 4, wherein BX represents a halogen- or sulfonic acid-substituted phenol or naphthol or aniline or naphthylamine.

6. Method as claimed in claim 5, wherein the coupling substance BX represents 1-naphthol-4-sulfonic acid which during coupling cleaves off an SO₃H⁻ group.

7. Method as claimed in claim 5, wherein the coupling substance BX represents 2,4,6-trihalogen-3-hydroxybenzoic acid or 2,4-dihalogen-1-naphthol or 4-monohalogen phenol or 4-monohalogen-1-naphthol which cleaves off a halogenide ion.

8. Method as claimed in claim 6, wherein the group X⁻ is measured potentiometrically.

9. Method as claimed in claim 7, wherein the group X⁻ is measured amperometrically.

10. Means for the simultaneous colorimetric and electrochemical measurement of an analyte containing an oxidizing enzyme and a chromogen A which accepts electrons from the enzyme wherein in addition to the chromogen A, which is an electron-rich aromatic nitroso compound, it contains a substance BX, the chromogen A being capable after reduction of reacting to form a coloured substance A'B by coupling with the substance BX with cleavage of an electrochemically measurable group X⁻ and wherein the agent additionally contains a sensor electrode for the electrochemical measurement of the presence or concentration of the cleaved group X⁻.

11. Means as claimed in claim 10, wherein the substance BX represents a phenol or naphthol compound or aniline or naphthylamine containing a readily substitutable, electrochemically measurable leaving group X⁻.

12. Means as claimed in claim 11, wherein the substance BX represents 1-naphthol-4-sulfonic acid.

13. Means as claimed in claim 11, wherein BX is 2,4-dihalogen-1-naphthol or 4-monohalogenphenol or 4-monohalogen-1-naphthol or 2,4,6-trihalogen-3-hydroxybenzoic acid.

14. Means as claimed in claims 10-12, wherein it additionally contains a mediator for electron transfer from the enzyme to the chromogen A.

15. Means as claimed in claims 10-14, wherein it additionally contains an optical measuring device to determine the presence or concentration of the coloured substance A'B.

## Revendications

1. Procédé de mesure colorimétrique et électrochimique simultanées d'un analyte avec une enzyme oxydante et un chromogène A qui accepte des électrons de l'enzyme, caractérisé en ce que le chromogène A est un composé nitroso aromatique riche en électrons qui, après la réduction en un composé imino A' apte au couplage, réagit par couplage avec une substance BX pour donner une substance colorée A'B, dont la concentration est mesurée par colorimétrie à titre de mesure de la présence ou de la quantité de l'analyte et en ce que, lors de la réaction de couplage, est libéré un groupe X⁻ mesurable par électrochimie, dont la concentration est mesurée par électrochimie à titre de mesure de la quantité de l'analyte.

2. Procédé selon la revendication 1, caractérisé en ce que le chromogène représente un composé répondant à la Formule I 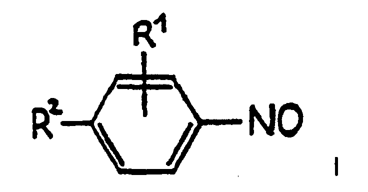
où R¹
représente un atome d'hydrogène, un groupe hydroxyle, un groupe alkyle, un groupe alkyle substitué par un groupe hydroxyle, un groupe COOH, un groupe PO₃H₂ ou un groupe SO₃H, un groupe alcoxy, un groupe alkylthio, un groupe aryloxy, un groupe arylthio, un atome d'halogène, un groupe amino ou un groupe amino qui est substitué une ou plusieurs fois par un groupe alkyle ou par un groupe alkyle substitué par un groupe hydroxyle, un groupe PO₃H₂, un groupe dialkylphosphinyle, un groupe SO₃H ou un groupe CO₂H,
et R²
représente un groupe hydroxyle, un groupe alcoxy, un groupe aryloxy, un groupe arylthio ou un groupe alkylthio, le résidu alkyle étant non-substitué ou substitué à son tour par un groupe hydroxyle, un groupe alcoxy, un groupe amino ou un groupe amino substitué une ou plusieurs fois par un groupe alkyle, un groupe PO₃H₂, un groupe SO₃H ou un groupe CO₂H en tant que tels ou sous forme saline en tant que sels d'ammonium, de métal alcalin ou de métal alcalino-terreux, ou un groupe amino NR³R⁴, dans lequel R³ et R⁴ peuvent être identiques ou différents et représentent un atome d'hydrogène, un groupe aryle ou un groupe alkyle, qui peuvent être substitués à leur tour par un groupe hydroxyle, un groupe alcoxy, un groupe hydroxyalcoxy, un groupe polyalcoxy ou un groupe polyalcoxy substitué, un groupe PO₃H₂, un groupe SO₃H, un groupe COOH en tant que tels ou sous forme saline ou par un groupe amino ou un groupe amino substitué une ou plusieurs fois par un groupe alkyle, ou dans lequel R³ et R⁴ peuvent représenter un résidu alkylène ou un résidu alkylène qui est interrompu par un atome d'oxygène, un atome de soufre ou un atome d'azote, l'atome d'azote étant substitué par un groupe alkyle, un groupe hydroxyalkyle, un groupe hydroxyalcoxyalkyle, un groupe alcoxyhydroxyalkyle, un groupe alcoxycarbonylalkyle, un groupe dioxanylylalkyle ou un groupe polyalcoxyalkyle, qui peut à son tour être non-substitué ou substitué dans la partie alkyle par un résidu hydroxyle, ou lorsque R¹ se trouve en position ortho par rapport à NR³R⁴, R³ ou R⁴ peuvent également représenter ensemble avec R¹ un résidu alkylène.

3. Procédé selon la revendication 1, caractérisé en ce que le chromogène représente un composé répondant à la Formule générale II, dans laquelle 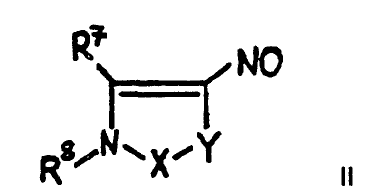
X-Y représente un groupe NR⁵-CO ou un groupe N=CR⁶
R⁵ représente un atome d'hydrogène, un groupe alkyle, un groupe alkyle, qui peut être substitué par un groupe hydroxyle, un groupe carboxyle, un groupe SO₃H, un groupe PO₃H₂, un groupe dialkylphosphinyle
R⁶ représente un atome d'hydrogène, un groupe alkyle, un groupe alcényle, un groupe alcoxy, un groupe alkylthio, un groupe aryle, un groupe arylthio, un groupe arylalkyle, qui peuvent être substitués respectivement par un groupe hydroxyle, un groupe carboxyle, un groupe SO₃H, un groupe PO₃H₂, un sel d'un de ces résidus acides et/ou un groupe alcoxycarbonyle ; ou
un groupe amino ou un groupe amino qui est substitué par un ou deux résidus alkyles qui peuvent porter un ou plusieurs résidus hydroxyles, carboxyles et/ou alcoxycarbonyles, où lorsque le groupe amino est substitué par deux résidus alkyles, ces résidus peuvent également se refermer en un cycle qui peut être interrompu outre par l'atome N du groupe amino, également par un atome d'oxygène, un atome de soufre ou un atome d'azote supplémentaire, ou un groupe amino ou un groupe amino qui est substitué par un ou deux groupes acyles, alcoxy et/ou arylalcoxycarbonyles, un groupe H₂N-CO, un groupe alkyle, un groupe arylalkyle et/ou un groupe arylcarbamoyle ; ou
un groupe carboxyle, un groupe alcoxycarbonyle, un groupe carboxamido ou un atome d'halogène, et
R⁷ représente un groupe alkyle, un groupe thioalkyle ou un groupe arylalkyle qui peut être substitué par un groupe hydroxyle, un groupe carboxyle, un groupe SO₃H ou un groupe PO₃H₂ ou un groupe amino ou un groupe amino qui est substitué par un ou deux groupes alkyles qui peuvent à leur tour être substitués par un groupe hydroxyle, un groupe carboxyle, un groupe SO₃H, un groupe dialkylphosphinyle ou un groupe PO₃H₂, où au moins R⁶ et/ou R⁷ représentent un groupe amino, et
R⁸ représente un groupe alkyle ou un groupe arylalkyle qui peut être substitué par un groupe hydroxyle, un groupe carboxyle, un groupe SO₃H ou un groupe PO₃H₂.

4. Procédé selon les revendications 1 à 3, caractérisé en ce que la substance apte au couplage BX représente un composé aromatique avec un substituant X facilement substituable par une réaction de substitution aromatique nucléophile.

5. Procédé selon la revendication 4, caractérisé en ce que BX représente du phénol ou du naphtol ou de l'aniline ou de la naphtylamine substitué(e) par un atome d'halogène ou un groupe d'acide sulfonique.

6. Procédé selon la revendication 5, caractérisé en ce que la substance apte au couplage BX représente de l'acide 1-naphtol-4-sulfonique qui libère lors du couplage un groupe SO₃H⁻.

7. Procédé selon la revendication 5, caractérisé en ce que la substance apte au couplage BX représente de l'acide 2,4,6-trihalogéno-3-hydroxybenzoïque ou du 2,4-dihalogéno-1-naphtol ou du 4-monohalogénophénol ou -1-naphtol, qui libère un ion d'halogénure.

8. Procédé selon la revendication 6, caractérisé en ce que le groupe X⁻ est mesuré par potentiométrie.

9. Procédé selon la revendication 7, caractérisé en ce que le groupe X⁻ est mesuré par ampérométrie.

10. Moyen pour la mesure colorimétrique et électrochimique simultanées d'un analyte contenant une enzyme oxydante et un chromogène A, qui accepte des électrons de l'enzyme, caractérisé en ce qu'il contient outre le chromogène A qui représente un composé nitroso aromatique riche en électrons, une substance BX, le chromogène A étant capable, après la réduction par le couplage avec la substance BX, en libérant un groupe X⁻ mesurable par électrochimie, de réagir pour donner une substance A'B et en ce que le moyen contient en outre une électrode détectrice pour la mesure électrochimique de la présence ou de la concentration du groupe X⁻ libéré.

11. Moyen selon la revendication 10, caractérisé en ce que la substance BX représente un composé phénol ou naphtol ou aniline ou naphtylamine avec un groupe de clivage X⁻ facile à substituer et mesurable par électrochimie.

12. Moyen selon la revendication 11, caractérisé en ce que la substance BX représente l'acide 1-naphtol-4-sulfonique.

13. Moyen selon la revendication 11, caractérisé en ce que BX est le 2,4-dihalogéno-1-naphtol ou le 4-monohalogénophénol ou -1-naphtol ou l'acide 2,4,6-trihalogéno-3-hydroxybenzoïque.

14. Moyen selon les revendications 10 à 12, caractérisé en ce qu'il contient en outre un médiateur pour le transfert d'électrons de l'enzyme sur le chromogène A.

15. Moyen selon les revendications 10 à 14, caractérisé en ce qu'il contient en outre un dispositif de mesure optique pour la détermination de la présence ou de la concentration de la substance colorée A'B.
